# FASCICULE DE BREVET EUROPEEN

(11) **EP 3 914 298 B1**
(45) Date de publication et mention de la délivrance du brevet: **12.11.2025**
(21) Numéro de dépôt: 20705243.2
(22) Date de dépôt: 24.01.2020
(51) Int. Cl.: A61K 47/69, A61K 51/06, A61M 1/16, A61M 1/36, G01N 33/48

(54) **DISPOSITIF D'EXTRACTION CONJOINTE D'UN CATION MÉTALLIQUE ET D'UNE MOLÉCULE CIBLE**
VORRICHTUNG ZUR GEMEINSAMEN EXTRAKTION EINES METALLKATIONS UND EINES ZIELMOLEKÜLS
DEVICE FOR JOINT EXTRACTION OF A METAL CATION AND A TARGET MOLECULE

(30) Priorité: 25.01.2019 FR 1900699
(43) Date de publication de la demande: 01.12.2021
(73) Titulaire: Mexbrain, 69100 Villeurbanne (FR); UNIVERSITE CLAUDE BERNARD - LYON 1, 69100 Villeurbanne (FR); CENTRE NATIONAL DE LA RECHERCHE SCIENTIFIQUE (CNRS), 75016 Paris (FR)
(72) Inventeur: TILLEMENT, Olivier, 69270 Fontaines Saint-Martin (FR); LUX, François, 69003 Lyon (FR); VICTOR, Marie, 69007 Lyon (FR); BECHET, Denise, 69004 Lyon (FR); NATUZZI, Marco, 69100 Villeurbanne (FR); TILLEMENT, Jules, 69270 Fontaines Saint-Martin (FR); BRICHART, Thomas, 69006 Lyon (FR)
(74) Mandataire: Plasseraud IP
(86) Numéro de dépôt international: PCT/FR2020/050104
(87) Numéro de publication internationale: WO 2020/152426

(56) Documents cités:
- US-A1- 2011 158 986
- US-A1- 2013 102 553
- US-A1- 2013 108 549
- US-A1- 2017 209 604

## Description

### Domaine technique

La présente invention concerne le domaine des dispositifs médicaux, plus particulièrement les dispositifs permettant d'extraire conjointement au sein d'un organisme au moins un cation métallique et au moins une molécule cible. L'utilisation de ces dispositifs permet par exemple de prévenir et/ou traiter des pathologies liées à une dérégulation de l'homéostasie des métaux et/ou de molécules cibles dans l'organisme, par exemple les maladies neurologiques et/ou protéinopathies.

### Technique antérieure

Le maintien de l'homéostasie du milieu intérieur de l'organisme, c'est-à-dire de l'ensemble des liquides ou fluides biologiques de l'organisme, est nécessaire au bon fonctionnement de ce dernier. Dans de nombreuses pathologies, des dysrégulations systémiques ou locales de l'homéostasie des métaux et/ou de peptides ou protéines ont été mises en évidence.

En ce qui concerne les métaux, des thérapies de chélation, visant à diminuer la concentration en ions métalliques sont déjà utilisées depuis de nombreuses années dans les cas d'intoxications aigues. Ainsi, un certain nombre de chélatants sont déjà acceptés chez l'homme, chacun étant associé à un groupe de métaux particuliers (G. Crisponi et al., Coordination Chemistry Reviews, 2015).

De plus en plus d'études scientifiques mettent en avant le rôle important que pourraient avoir les métaux dans nombre d'atteintes neurologiques, notamment le fer, mais également le cuivre, le zinc, le manganèse et même l'aluminium et le plomb (E. J. McAllum et al., J. Mol. Neurosci., 2016). C'est en particulier le cas pour la neurodégénérescence avec surcharge en fer qui est une maladie rare associée à une anomalie génétique liée à une accumulation du fer dans certaines zones du cerveau et qui ne bénéficie à ce jour que de traitements palliatifs (S. Wiethoff et al., Handb. Clin. Neurol., 2017). Par ailleurs, de nombreuses études ont montré que le fer avait tendance à s'accumuler dans le cerveau avec l'âge (J. Acosta-Cabronero et al., Journal of Neuroscience, 2016). La maladie de Wilson est aussi une maladie génétique entrainant une accumulation de cuivre dans l'organisme et entrainant différents problèmes en particulier hépatiques et/ou neurologiques (Anna Cz 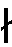 onkowskal et al., Nature Rev., 2018).

Plusieurs maladies neurologiques telles qu'Alzheimer, Parkinson et la maladie d'Huntington s'accompagnent également d'accroissement de la quantité de fer dans des zones spécifiques entrainant des dommages cellulaires ainsi que du stress oxydant (A. A. Belaidi et al., Journal of Neurochemistry, 2016). A titre d'exemple, la maladie de Huntington est une maladie neurodégénérative se traduisant par des troubles du mouvement, un déclin cognitif et des problèmes d'ordre psychiatriques. Dans cette pathologie, de nombreux marqueurs du stress oxydant sont observés au niveau du cerveau qui peut être relié à une dérégulation de l'homéostasie du fer (S. J. A. van den Bogaard et al., International Review of Neurobiology, 2013). L'augmentation du niveau de fer au niveau de plusieurs régions du cerveau (putamen, noyau caudé et pallidum) a été ainsi validée par plusieurs études IRM dont celle de Bartzorkis (G. Bartzorkis et al., Archives of Neurology, 1999).

Dans ces mêmes pathologies, l'homéostasie d'autres composés biologiques est également perturbée. Dans la maladie d'Alzheimer, par exemple, le peptide A-ß(amyloid-beta), peptide de 42 acides aminés environ (39 à 43) s'accumule en formant des agrégats bêta-amyloïdes. Il a ainsi été proposé des traitements des maladies amyloïdes par extraction du peptide Aß des fluides biologiques (US2013/0045216 A1 ; M. Menendez-Gonzalez et al., Hypothesis and Theory, 2018). Toujours dans le but de traiter ou de ralentir le développement de la maladie d'Alzheimer, il a également été proposé une dilution du liquide cérébrospinal par remplacement et filtration de ce liquide, pour diminuer les taux de peptide Aß et de protéine Tau anormalement phosphorylées (phospho-Tau) (M.M. Gonzalez, Cureus, 2017).

Outre dans la maladie d'Alzheimer, il a été mis en évidence dans de nombreuses autres pathologies amyloïdes telles que la maladie de Parkinson et la maladie du prion, une conversion conformationnelle de protéines solubles normales en protéines insolubles, conduisant à la formation de plaques ou fibrilles amyloïdes. Ainsi, des anticorps ou de petites molécules ciblant spécifiquement ces protéines sont étudiés dans le but d'inhiber les étapes clés du processus d'agrégation de ces protéines anormales, réduire la conversion de protéines dans leur conformation pathologiques, réduire la toxicité des protéines pathologiques ou augmenter la clairance sélective des protéines anormales (N. Cremadeset al, Neurobiol Dis., 2018).

De plus, dans la maladie d'Alzheimer notamment, il a été mis en évidence des interactions entre certains ions métalliques, notamment des ions issus de métaux tels le zinc, le fer ou le cuivre, avec les peptides Aß pouvant conduire à une agrégation accrue des protéines (Tougu et al. Metallomics, 2010).

Il est ainsi admis que dans de nombreuses protéinopathies, les cations métalliques jouent un rôle important dans la formation de configurations anormales de certaines protéines, en particulier certains favorisent la formation d'agrégats, fibrilles ou autres dépôts solides. Dans les protéinopathies, on serait ainsi localement dans une double dérégulation de l'homoéostasie, dérégulation de l'homéostasie de certains métaux et dérégulation de l'homéostasie de molécules cibles de type protéines, à l'origine des agrégats et autres dépôts solides.

Bien que les connaissances scientifiques relatives à ces différentes pathologies progressent (Pfaender S et al., 2014; Boland B et al., 2018 ; ladanza MG et al., 2018), il n'existe à ce jour aucun traitement efficace de la maladie d'Alzheimer ou de la maladie de Parkinson et, plus généralement, des maladies neurodégénératives et plus globalement des maladies impliquant de multiples dérégulations à l'origine d'une dyshoméostasie. Le document US2011/0158986 est considéré comme faisant partie de l'art antérieur.

### Problème technique

A ce jour, il existe donc un besoin de développer de nouveaux moyens permettant de prévenir et/ou traiter des pathologies impliquant de multiples dérégulations à l'origine d'une dyshoméostasie et la formation de conformations protéiques aberrantes conduisant à la formation de dépôts, agrégats, fibrilles ou plaques comprenant lesdites protéines. Ces moyens présenteraient ainsi un ou plusieurs des avantages suivants :
- une extraction ciblée et combinée de ces composés (métaux et protéines cibles) au sein de l'organisme, que ces derniers soient présents en forte ou faible quantité,
- une absence de toxicité,
- lorsque ces composés sont co-agrégés dans l'organisme, une absence de relargage de l'un ou de l'autre composé isolément pouvant intervenir lors du processus d'extraction,
- une durée d'efficacité locale déterminée et non liée à la biodistribution d'un médicament administré,
- une action locale même accessible au-delà de la barrière hémato-encéphalique, dans le cas du traitement de maladies neurologiques,
- une application adaptée à la prévention et/ou au traitement de n'importe quelle pathologie liée à une dérégulation de l'homéostasie de composés cibles, notamment associant des métaux et des molécules cibles susceptibles de se co-agréger.

Ces avantages et bien d'autres sont décrits dans la présente divulgation.

### Exposé de l'invention

Il est proposé un dispositif d'extraction conjointe d'au moins un cation métallique et d'au moins une molécule cible d'un fluide biologique, d'un agrégat biologique, d'un organe ou d'un tissu à des fins diagnostiques ou thérapeutiques, caractérisé en ce qu'il comprend :
a. au moins un ligand présentant une affinité spécifique pour la molécule cible;
b. au moins. un moyen permettant l'extraction du cation métallique, ledit moyen étant un fluide de perfusion comprenant au moins un agent chélatant, ledit fluide de perfusion étant contenu dans un système de dialyse ou de microdialyse.

Il est proposé également, un système de microdialyse comprenant ledit dispositif d'extraction et tel que ledit système comprend au moins :
- un réservoir de perfusion 7 comprenant le fluide de perfusion 11 et un réservoir de recueil 8 comprenant le fluide de perfusion comprenant les composés extraits 12; ou un réservoir mixte 22 comprenant le fluide de perfusion 11, 12 ;
- un cathéter bidirectionnel 3 connectant la sonde de microdialyse 1 au réservoir de perfusion 7 et au réservoir de recueil 8 ou au réservoir mixte 22 ;
- une sonde de microdialyse 1 comprenant un premier lumen 4 permettant le passage du fluide de perfusion 11 vers un second lumen 5, ledit second lumen 5 permettant d'évacuer le fluide de perfusion comprenant les composés extraits 12 et une membrane de microdialyse 2 comprise entre le second lumen 5 et l'extérieur de la sonde de microdialyse 1 en contact avec le fluide biologique.

Selon un mode de réalisation alternatif, il est proposé un système de dialyse comprenant ledit dispositif d'extraction et tel qu'il comprend au moins:
- une sonde à lumens séparés 17, comprenant un second cathéter 5bis permettant l'entrée du fluide biologique dans le système de dialyse et un premier cathéter 4bis permettant la sortie du fluide biologique du système de dialyse;
- un réservoir 23 comprenant i) le fluide de perfusion 11; ii) un compartiment de dialyse comprenant une membrane de dialyse 18 séparant le fluide de perfusion 11 du fluide biologique, le fluide biologique entrant dans ledit compartiment de dialyse via le second cathéter 5bis et en sortant via le premier cathéter 4bis.

Les caractéristiques exposées dans les paragraphes suivants peuvent, optionnellement, être mises en œuvre. Elles peuvent être mises en œuvre indépendamment les unes des autres ou en combinaison les unes avec les autres.

Le moyen d'extraction du cation métallique peut être un fluide de perfusion utilisé dans un système de dialyse ou de microdialyse, ledit fluide de perfusion comprenant en outre ledit ligand présentant une affinité spécifique pour la molécule cible.

Avantageusement, la constante de complexation log(KC1) de l'agent chélatant pour au moins un cation métallique est supérieure à 10, de préférence, supérieure ou égale à 15 et ledit au moins un cation est choisi parmi les cations des métaux Cu, Fe, Zn, Hg, Cd, Pb, Mn, Co, Gd et Al, pris seul ou en association et plus particulièrement Cu, Fe et Zn, pris seul ou en association.

Selon un mode de réalisation préféré, le moyen d'extraction permet d'extraire les cations d'un fluide biologique, d'un agrégat biologique, d'un organe ou d'un tissu lorsque la teneur desdits cations métalliques est inférieure à 1 ppm, préférentiellement inférieure à 0,1 ppm, plus préférentiellement inférieure à 0,01ppm et, plus préférentiellement encore, inférieure à 1 ppb.

Le moyen d'extraction peut permettre d'extraire une quantité de cations métalliques représentant au moins 1% de sa masse et, de préférence, plus de 10% de sa masse.

Le dispositif est avantageusement un fluide de perfusion compris dans un système de dialyse comprenant une membrane de dialyse et un réservoir comprenant le fluide de perfusion, ledit fluide de perfusion étant choisi parmi :
- une solution de nanoparticules comprenant à titre de principe actif au moins un agent chélatant et une solution d'au moins un ligand présentant une affinité spécifique pour la molécule cible, le diamètre moyen desdites nanoparticules et celui du ligand étant supérieurs aux pores de la membrane de dialyse. ou de microdialyse,
- une solution de polymères, lesdits polymères étant greffés à au moins un principe actif qui est un agent chélatant et une solution d'au moins un ligand présentant une affinité spécifique pour la molécule cible, le diamètre moyen est supérieur aux pores de ladite membrane de dialyse ou de microdialyse.

Préférentiellement, le dispositif comprend un système de dialyse comprenant une membrane de dialyse et un réservoir comprenant un fluide de perfusion, ledit fluide de perfusion étant choisi parmi :
- une solution de nanoparticules comprenant à titre de principe actif au moins un agent chélatant et une solution d'au moins un ligand présentant une affinité spécifique pour la molécule cible, le diamètre moyen desdites nanoparticules et celui du ligand étant supérieurs aux pores de la membrane de dialyse,
- une solution de polymères, lesdits polymères étant greffés à au moins un principe actif qui est un agent chélatant et une solution d'au moins un ligand présentant une affinité spécifique pour la molécule cible, le diamètre moyen est supérieur aux pores de ladite membrane de dialyse.

Selon un mode de réalisation avantageux, les agents chélatants sont obtenus par greffage sur les nanoparticules ou sur le polymère de l'une des molécules complexantes suivantes ou ses dérivés : DOTA, DTPA, EDTA, EGTA, BAPTA, NOTA, DOTAGA, DFO, DOTAM, NOTAM, DOTP, NOTP, TETA, TETAM, TETP et DTPABA, ou leurs mélanges.

Préférentiellement, les nanoparticules sont des nanoparticules à base de polysiloxane d'un diamètre moyen compris entre 3 et 50 nm, comprenant l'agent chélatant obtenu par greffage de DOTA, DOTAGA, EDTA ou du DTPA sur les nanoparticules.

Selon un mode de réalisation particulier, l'agent chélatant contient au moins un cation alcalino-terreux, préférentiellement un cation de métaux choisis parmi Ca et Mg.

Avantageusement, au moins 10% des agents chélatants dudit dispositif sont pré-complexés par un cation alcalino-terreux, préférentiellement 20%, plus préférentiellement 30% et plus préférentiellement encore plus de 50% des agents chélatants dudit dispositif sont pré-complexés par un cation alcalino-terreux.

Selon un mode de réalisation, les nanoparticules ou les polymères, comprenant l'agent chélatant obtenu par greffage de DOTA, DOTAGA, EDTA ou DTPA, ont un diamètre moyen supérieur à 20 kDa et inférieur à 1 MDa.

Avantageusement, les nanoparticules sont à base de polysiloxane ou les polymères sont à base de chitosane ou de polyéthylèneglycol oude polyvinylalcools.

Selon un mode de réalisation la molécule cible est choisie parmi les protéines, les peptides et les glycoprotéines. Avantageusement, la molécule cible est choisie parmi les protéines amyloïdogéniques et les composants des structures amyloïdes (sous forme native monomère, ou sous forme d'oligomères, ou des fibrilles ou d'agrégats ou de molécules responsables de leur formation ou de leur accumulation).

Selon un mode de réalisation préféré, la molécule cible est choisie parmi les molécules impliquées dans les amyloses, les tauopathies ou toute pathologie présentant un dépôt à base d'une ou plusieurs protéines. Avantageusement, la molécule cible est choisie parmi les protéines et/ou leurs précurseurs telles les chaînes d'immunoglobuline légères et lourdes, la protéine amyloïde sérique, la transthyrétine, l'apolipoprotéine AI, All, AVI, CII ou CIII, la microglobuline beta 2, la gelsoline, le lysosyme, le fibrinogène, la cystatine C, le facteur atrial natriurétique, la calcitonine, l'amyline, l'insuline, la prolactine, la lactoferrine, la cadhérine, A-Bri, A-Dan, le peptide beta amyloïde, la protéine prion, l'alpha-synucléine, la protéine Tau, la superoxydismutase, la huntingtin, la neuroserpine, l'actine, la ferritine ou leurs mélanges.

De façon préférée, le ligand est un anticorps ou un ligand protéique artificiel de la molécule cible. Avantageusement, le ligand est choisi parmi :
- les anticorps ciblant la protéine béta-amyloide, préférentiellement Solanezumab, Aducanumab, Crenezumab, Ponezumab, GSK933776, Gantenerumab, AAB-003, AAB-001, BAN2401, LY2599666, LY3002813, LY3372993, MEDI1814, SAR228810 ;
- les anticorps ciblant l'alpha-synucléine, préférentiellement BII054 ou PRX002 ;
- les anticorps ciblant la protéine tau, préférentiellement BII076, BII092, ABBV-8E12, JNJ-63733657, LY3303560, RG7345, RO7105705, UCB0107;
- les anticorps ciblant la protéine amyloïde sérique, préférentiellement : Dezamizumab ou GSK2398852, Miridesap ou GSK2315698, GSK3039294 ;
- les anticorps ciblant la transthyrétine, préférentiellement PRX004 ;
- les aptamères
- les ligands protéiques artificiels présentant une affinité spécifique pour l'une au moins des molécules, optionnellement choisis parmi : ABD, Adhiron, Adnectin, Affibody, Affilin, Affimer, Affitin, Alphabody, Anticalin, Armadillo repeat proteins, Atrimer/tetranectin, Avimer/Maxibody, Centyrin, DARPin1 ;
- les ligands protéiques artificiels de moins de 50 kDA, de préférence moins de 30 kDa et, plus préférentiellement de moins de 5kDa, greffées sur une nanoparticules ou un polymère de plus de 5 nm de diamètre hydrodynamique, préférentiellement de plus de 100 kDa, ou
- leurs mélanges.

Selon un mode de réalisation préféré, le dispositif est utilisé dans le traitement :
- des amyloses systémiques et/ou localisées ; notamment des amyloses choisies parmi : amylose de type AL (chaines légères d'immunoglobulines), amylose de type AH (chaines lourdes d'immunoglobulines, amylose AA (protéine amyloïde sérique), amylose ATTR (transthyrétine), cardiopathies amyloïdes, amyloses rénales, diabète de type II, maladies à Prion ou maladies liées à une protéine amyloïde ,
- des tauopathies notamment : des tauopathies choisies parmi : maladie d'Alzheimer, paralysie supranucléaire progressive, démence fronto-temporale ;
- des pathologies présentant un dépôt à base d'au moins une protéique;
- des maladies présentant une dyshoméostasie métallique notamment la maladie de Wilson ou des troubles neurologiques sans caractéristique amyloïde tels que l' autisme ou la schizophrénie, ...), ou des troubles neurologiques avec caractéristique amyloïde tels que les amyloses touchant ou non le système nerveux central et/ou périphérique.

### Brève description des dessins

D'autres caractéristiques, détails et avantages de l'invention apparaîtront à la lecture de la description détaillée ci-après, et à l'analyse des dessins annexés, sur lesquels :
**Fig. 1**

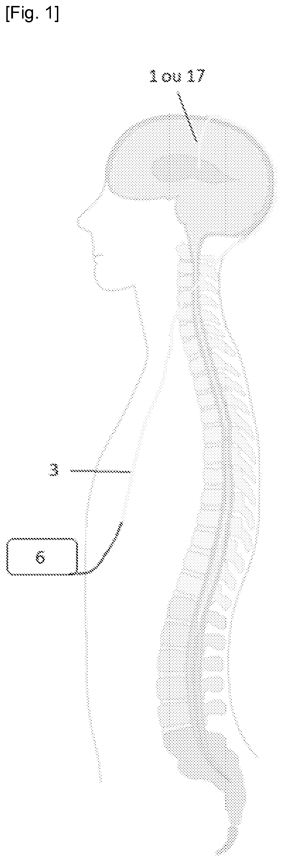

   [Fig. 1] montre un système de dialyse selon un mode de réalisation de l'invention; ledit système comprenant une sonde de dialyse 1, 17 placée au niveau cérébral. Dans un tel mode de réalisation, le fluide biologique est le liquide céphalorachidien.
**Fig. 2**

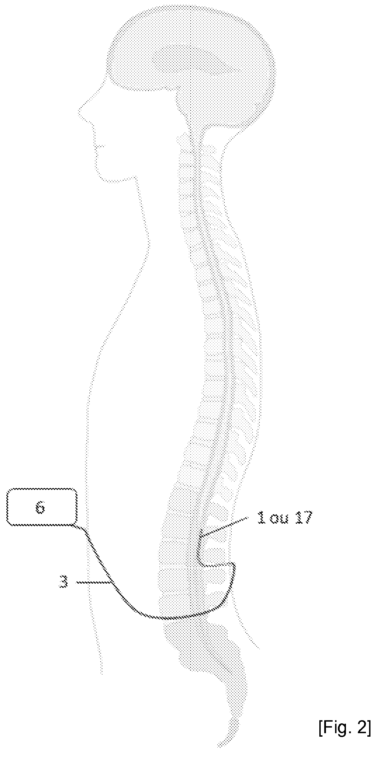

   [Fig. 2] montre un système de dialyse selon un autre mode de réalisation de l'invention; ledit système comprenant une sonde de dialyse ou de microdialyse 1, 17 placée au niveau rachidien dans liquide céphalo-rachidien. Dans un tel mode de réalisation, le fluide biologique est avantageusement le liquide rachidien.
**Fig. 3**

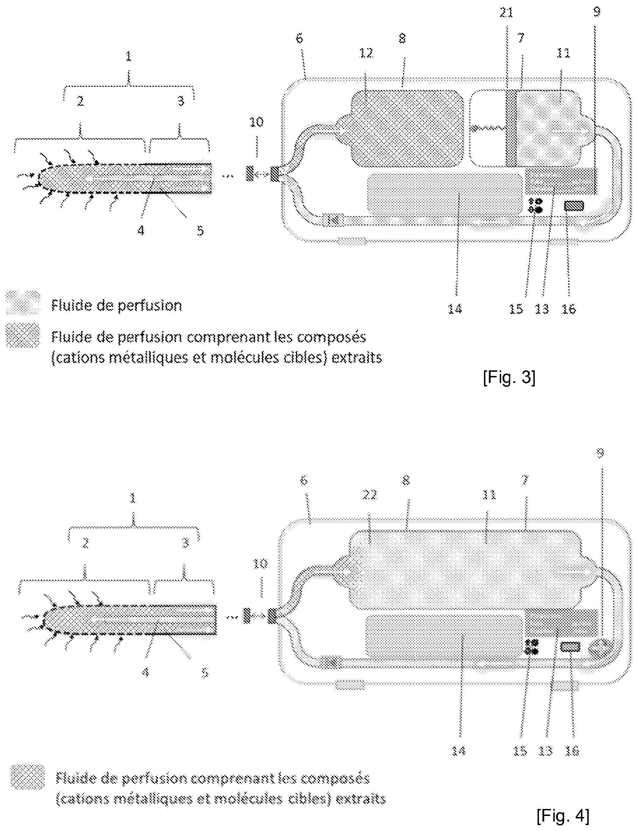

   [Fig. 3] montre un système de microdialyse selon un mode de réalisation de l'invention; ledit système comprenant i) une sonde de microdialyse 1 avec un premier lumen 4, un second lumen 5 et une membrane de microdialyse 2 entre le second lumen 4 et l'extérieur de la sonde de microdialyse 1 en contact avec le fluide biologique; ii) un réservoir de perfusion 7 comprenant le fluide de perfusion 11 et un réservoir de recueil 8 comprenant le fluide de perfusion comprenant les composés extraits 12; iii) un cathéter bidirectionnel 3 connectant la sonde de microdialyse 1 aux réservoir de perfusion 7 et de recueil 8 contenus dans un boitier 6 de microdialyse.
**Fig. 4**
   [Fig. 4] montre un système selon un second mode de réalisation de l'invention; ledit système comprenant une sonde de microdialyse 1 ; un cathéter bidirectionnel 3 et un réservoir mixte 22. Ledit réservoir mixte 22 a pour fonction de contenir un volume donné de fluide de perfusion dont la composition en composés extraits (cations métalliques et molécules cibles) augmente au fur et à mesure des cycles de passage du fluide dans le système.
**Fig. 5**

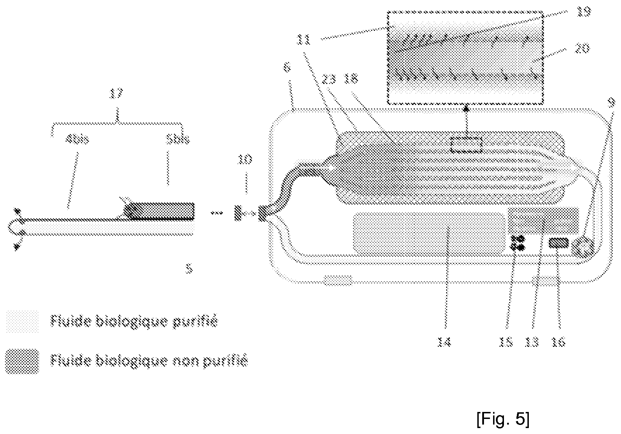

   [Fig. 5] montre un système de dialyse selon un troisième mode de réalisation; ledit système comprenant au moins:
   - une sonde à lumens séparés 17, comprenant un second cathéter 5bis permettant l'entrée du fluide biologique dans le système et un premier cathéter 4bis permettant la sortie du fluide biologique du système;
   - un réservoir de fluide d'extraction 23 comprenant
      i) un fluide de perfusion 11;
      ii) un compartiment de dialyse comprenant une membrane de dialyse 18 séparant le fluide de perfusion 11 du fluide biologique, le fluide biologique entrant dans ledit compartiment de dialyse via le second cathéter 5bis et en sortant via le premier cathéter 4bis.

### Description détaillée

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

Les inventeurs ont développé un dispositif médical permettant l'extraction conjointe d'au moins un cation métallique et d'au moins une molécule cible, préférentiellement au moins deux molécules cibles, d'un fluide, d'un agrégat biologique, d'un organe ou d'un tissu à des fins diagnostiques ou thérapeutiques.

On entend par « extraction conjointe d'au moins un cation métallique et d'au moins une molécule cible», l'extraction simultanée dudit cation métallique et de ladite molécule cible ou l'extraction successive dudit cation métallique et de ladite molécule cible dans quelque ordre que ce soit, l'extraction de ces deux composés étant effectuée avec un court laps de temps entre les deux extractions, c'est-à-dire préférentiellement moins de 24h, plus préférentiellement moins de 12h, plus préférentiellement encore moins d'1 heure.

On entend par « un cation métallique », au moins un cation métallique. S'il s'agit de plusieurs cations métalliques, il peut s'agir de cations métalliques de même nature ou de natures différentes.

On entend par « une molécule cible », au moins une molécule cible. S'il s'agit de plusieurs molécules cibles, il peut s'agir de molécules cibles de même nature ou de natures différentes.

On entend par « ligand », une molécule qui se lie, préférentiellement de manière réversible, à la molécule cible de façon spécifique. Avantageusement, la liaison spécifique ligand - molécule cible s'effectue grâce aux forces entre molécules, telles que les liaisons ioniques, les liaisons d'hydrogène, les interactions hydrophobe set les forces de van der Waals. Ainsi, l'interaction ligand - molécule cible est réversible et plus ou moins forte suivant le nombre et la nature des liaisons formées. La force de cette interaction est définie par l'affinité pour la molécule cible, celle-ci étant liée à la constante de dissociation.

L'extraction desdits composés biologiques, c'est-à-dire dudit cation métallique et de ladite molécule cible, a pour objectif le maintien de l'homéostasie en ces dits composés à des fins thérapeutiques ou diagnostiques. Le maintien de l'homéostasie s'entend de la régulation de la teneur en ces dits composés au sein d'un organisme, notamment dans le but d'extraire ces dits composés en excès qui peuvent être responsables de pathologies. Lesdits composés peuvent être en excès au sein d'un fluide biologique ou d'un agrégat biologique. L'extraction d'un des composants peut également avoir aussi pour objectif de passer la concentration d'au moins un des composés biologiques en dessous du seuil de solubilité des agrégats biologiques liés à la pathologie et diminuer ainsi leur formation et/ou entrainant leur dissolution.

Par « fluide biologique », on entend tout fluide produit par l'organisme auquel il se rapporte. Il peut s'agir, d'un fluide circulant ou non. Plus particulièrement, il peut s'agir du sang, de la lymphe, de la moëlle osseuse, du chyle, de tout fluide interstitiel, du liquide céphalo-rachidien (LCR) ou plus spécifiquement le liquide cérébrospinal ou le liquide rachidien, le liquide synovial, le liquide péritonéal.

Par « agrégat biologique », on entend toute accumulation de molécules cibles et/ou de métaux sous forme de fibrilles, de composés matriciels ou de plaques. A titre d'exemple, il peut s'agir de composants amyloïdes par exemple sous forme de fibrilles ou de plaques, d'accumulations de protéines Tau, de plaques graisseuses telles que notamment les plaques d'athérome, etc.

Selon l'invention, le terme « organe » s'entend de tous les organes avec lesquels le dispositif de l'invention peut être mis en contact ou à l'intérieur duquel ledit dispositif peut être implanté ou inséré. Préférentiellement le ou les organes sont choisis parmi le cerveau, le foie, le pancréas, les intestins et les poumons.

Selon l'invention, le terme « tissu » s'entend de tous les tissus avec lesquels le dispositif de l'invention peut être mis en contact ou à l'intérieur duquel ledit dispositif peut être implanté ou inséré. Préférentiellement le ou les tissus sont choisis parmi le péritoine et le tissu tumoral (le cas échéant d'une tumeur). Par exemple, ledit dispositif peut être mis en contact, inséré ou implanté par endoscopie, notamment au sein d'une tumeur.

Par « au moins un(e) », on entend un ou plusieurs des composés en question, de même nature ou de nature différentes.

Par « dialyse », on entend également les dialyses particulières telles que, par exemple, les microdialyses.

Le dispositif d'extraction comprend un ligand présentant une affinité spécifique pour la molécule cible. Ainsi ledit composé est apte à se lier de façon spécifique à ladite molécule cible. Il peut s'agir d'un anticorps, d'un nanobody, d'un peptide, d'une protéine ou de tout autre ligand apte à se lier spécifiquement à la molécule cible.

On entend par « anticorps », une immunoglobuline formée de 4 chaines polypeptidiques, deux lourdes H et deux légères L, capable de lier spécifiquement un antigène, appelé également molécule cible dans le cadre de la présente invention.

On entend par « nanobody » un élément d'anticorps capable de lier spécifiquement un antigène ou molécule cible dans le cadre de la présente invention.

On entend « ligand protéique artificiel » (« scaffold protein » ou « engineered protein » en anglais) un composé ou des fragments de protéines sélectionné(s) pour leur affinité envers des molécules cibles spécifiques. Ils sont généralement plus légers que les anticorps, souvent plus faciles à produire également et stables chimiquement. Avantageusement, les ligands protéiques artificiels sont. de moins de 50 kDA, de préférence moins de 30 kDa et, plus préférentiellement de moins de 3kDa. De tels ligands présentent une bonne surface spécifique. Ces ligands protéiques artificiels peuvent être choisis parmi : ABD, Adhiron, Adnectin, Affibody, Affilin, Affimer, Affitin, Alphabody, Anticalin, Armadillo repeat proteins, Atrimer/tetranectin, Avimer/Maxibody, Centyrin, DARPin1.

Le dispositif d'extraction comprend en outre un moyen permettant l'extraction d'au moins un cation métallique, ledit moyen étant un fluide de perfusion comprenant au moins un agent chélatant, ledit fluide de perfusion étant contenu dans un système de dialyse.

Selon l'invention, le terme « agent chélatant » s'entend d'un groupement organique capable de complexer au moins un cation métallique. La réaction de complexation peut être une transmétallation, c'est-à-dire un échange de deux cations métalliques. Dans un tel cas, l'agent chélatant peut être pré-complexé avec un premier cation métallique qui sera échangé par la suite avec le cation métallique cible.

Dans un mode de réalisation avantageux, au moins 10% des agents chélatants dudit dispositif sont pré-complexés par un cation alcalino-terreux, préférentiellement 20%, plus préférentiellement 30% et plus préférentiellement encore plus de 50% des agents chélatants dudit dispositif sont pré-complexés par un cation alcalino-terreux.

Selon un mode de réalisation préféré, la constante de complexation log(KC1) dudit agent chélatant pour au moins un desdits cations métalliques est supérieure à 10, notamment 11, 12, 13, 14, 15 et est de préférence supérieure ou égale à 15. Lorsque l'agent chélatant est pré-complexé avec un premier cation métallique la constante de complexation log(KC1') pour le premier cation métallique est inférieure à la constante de complexation log(KC1) du cation métallique cible.

Avantageusement l'agent chélatant complexe avec une constante au moins supérieure ou égale à 10 et de préférence supérieure ou égale à 15 au moins un des cations des métaux Cuivre (Cu), Fer (Fe), Zinc (Zn), Mercure (Hg), Cadmium (Cd), Plomb (Pb), Aluminium (Al), Manganèse (Mn), Arsenic (As), Mercure (Hg), Cobalt (Co), Nickel (Ni), Vanadium (V), Tungstène (W), Zirconium (Zr), Titane (Ti), Chrome (Cr), Argent (Ag), Bismuth (Bi), Etain (Sn), Scandium (Sc), Yttrium (Y), Lanthane (La), Cérium (Ce), Praséodyme (Pr), Néodyme (Nd), Samarium (Sm), Europium (Eu), Gadolinium (Gd), Terbium (Tb), Dysprosium (Dy), Holmium (Ho), Erbium (Er), Thulium (Tm), Ytterbium (Yb), Lutécium (Lu), Actinium (Ac), Uranium (U), Plutonium (Pu), Américium (Am) pris seul ou en association. Encore plus avantageusement, l'agent chélatant complexe au moins un des cations des métaux Cuivre, Fer, Zinc, Mercure, Cadmium, Plomb, Aluminium, Manganèse, et Gadolinium, notamment Manganèse et Gadolinium. De façon encore plus avantageuse, l'agent chélatant complexe au moins un des cations des métaux Cuivre, Fer et Zinc, pris seul ou en association.

Avantageusement la spécificité de l'agent chélatant pour ledit cation métallique à extraire est élevée par rapport aux autres oligoéléments cationiques, en particulier l'écart entre les constantes de complexation est de préférence supérieur à 3 et ; plus particulièrement, l'écart entre les constantes de complexation avec le calcium et le magnésium est de préférence supérieur à 3 et même supérieur à 5.

Selon un mode de réalisation préféré, ledit dispositif ou, plus spécifiquement le moyen permettant l'extraction d'au moins un cation métallique, contient également des oligoéléments, choisis parmi le calcium, magnésium, fer, cuivre, zinc et manganèse au sein du fluide de perfusion ou encore directement sur ledit agent chélatant. Dans ce dernier cas, il s'agit d'une réaction de transmétallisation et les cations sont spécifiquement choisis pour permettre une telle réaction. Un tel mode de réalisation permet par exemple de réguler l'homéostasie des métaux essentiels. L'agent chélatant contient au moins un cation alcalino-terreux, préférentiellement un cation de métaux choisis parmi Ca et Mg.

Selon un mode de réalisation de l'invention, le moyen d'extraction du cation métallique permet d'extraire ledit cation métallique d'un fluide biologique, d'un agrégat biologique, d'un organe ou d'un tissu lorsque sa teneur est inférieure à 1 ppm, notamment 0,1 ppm, 0,01ppm et est de préférence inférieure à 1 ppb.

Selon un mode de réalisation avantageux, ledit moyen d'extraction du cation métallique permet d'extraire une quantité de cations métalliques représentant au moins 1% de sa masse et, de préférence, plus de 10% de sa masse.

En outre, le dispositif d'extraction comprend au moins un ligand présentant une affinité spécifique pour une molécule cible. Ladite molécule cible est préférentiellement choisie parmi les protéines, les peptides, les glycoprotéines, plus préférentiellement elle est choisie parmi les composants des structures amyloïdes. Selon un mode de réalisation avantageux, la molécule cible comprend une séquence peptidique spécifique reconnue par le ligand.

Selon un mode de réalisation de l'invention, la molécule cible est choisie parmi les protéines, les peptides, les glycoprotéines.

Avantageusement, la molécule cible est choisie parmi les protéines amyloïdogéniques et les composants des structures amyloïdes, notamment sous forme native monomère ou sous forme d'oligomères, de fibrilles ou d'agrégats biologiques. La molécule cible peut également être une ou plusieurs molécules responsables de la formation ou de l'accumulation desdits oligomères, fibrilles ou agrégats biologiques.

Selon un mode de réalisation préféré, la molécule cible est choisie parmi les molécules impliquées dans les amyloses, les tauopathies ou toute pathologie présentant un dépôt à base d'une ou plusieurs protéines.

Selon un mode de réalisation compatible avec les modes précédents, la molécule cible est choisie parmi les protéines et/ou leurs précurseurs telles les chaînes d'immunoglobuline légères et lourdes, la protéine amyloïde sérique, la transthyrétine, l'apolipoprotéine AI, All, AVI, CII ou CIII, la microglobuline beta 2, la gelsoline, le lysosyme, le fibrinogène, la cystatine C, le facteur atrial natriurétique, la calcitonine, l'amyline, l'insuline, la prolactine, la lactoferrine, la cadhérine, A-Bri, A-Dan, le peptide beta amyloïde, la protéine prion, l'alpha-synucléine, la protéine Tau, la superoxydismutase, la huntingtin, la neuroserpine, l'actine, la ferritine ou leurs mélanges.

De façon préférée, le ligand est un anticorps ou un ligand protéique artificiel de la molécule cible. Avantageusement, le ligand est choisi parmi :
- les anticorps ciblant la protéine béta-amyloide, préférentiellement Aducanumab, Crenezumab, Ponezumab, GSK933776, Gantenerumab, AAB-003, AAB-001, BAN2401, LY2599666, LY3002813, LY3372993, MEDI1814, SAR228810,
- les anticorps ciblant l'alpha-synucléine, préférentiellement BII054 ou PRX002 ;
- des anticorps ciblant la protéine tau, préférentiellement BII076, BII092, ABBV-8E12, JNJ-63733657, LY3303560, RG7345, RO7105705, UCB0107;
- les anticorps ciblant la protéine amyloïde sérique, préférentiellement : Dezamizumab ou GSK2398852, Miridesap ou GSK2315698, GSK3039294 ;
- les anticorps ciblant la transthyrétine, préférentiellement PRX004 ;
- les aptamères ;
- les anticorps ciblant les molécules, notamment les protéines, les peptides et/ou leurs précurseurs, impliquées dans les amyloses, les tauopathies ou les pathologies présentant un dépôt à base protéique ; soit toutes les protéines et leurs précurseurs ;
- les ligands protéiques artificiels, avantageusement de moins de 50 kDA, de préférence moins de 30 kDa et, plus préférentiellement de moins de 3kDa, greffées sur une nanoparticules, préférentiellement une nanoparticule de polysiloxane, ou un polymère de plus de 5 nm de diamètre hydrodynamique, préférentiellement de plus de 100 kDa et, avantageusement, de moins de 1µm de diamètre hydrodynamique ou de moins de 1MDa. De tels ligands présentent une bonne surface spécifique. Dans ce mode de réalisation, il peut y en avoir une ou plusieurs protéines par nanoparticule ou par polymère. De plus, il est envisageable de greffer, sur la nanoparticule ou le polymère un ou plusieurs agents chélatants. Ces ligands protéiques artificiels peuvent être choisis parmi : ABD, Adhiron, Adnectin, Affibody, Affilin, Affimer, Affitin, Alphabody, Anticalin, Armadillo repeat proteins, Atrimer/tetranectin, Avimer/Maxibody, Centyrin, DARPin1, ou
- leurs mélanges.

Selon un mode de réalisation, le dispositif d'extraction comprend un fluide de perfusion comprenant au moins un agent chélatant, ledit fluide de perfusion étant contenu dans un système de dialyse ou de microdialyse ou tout dispositif miniaturisé de dialyse, en particulier à réservoir d'échange fixe.

Avantageusement et selon un mode de réalisation préféré, le système de dialyse ou microdialyse comprend :
a. une membrane de dialyse 18 ou de microdialyse 2 semi-perméable,
b. un ou plusieurs réservoirs 7/8 ou 22 ou 23 comprenant le fluide de perfusion.

Selon un mode de réalisation, ledit moyen d'extraction du cation métallique est un fluide de perfusion utilisé dans un système de dialyse ou de microdialyse qui comprend en outre le ligand présentant une affinité spécifique pour la molécule cible.

Selon l'invention, le terme « système de dialyse » s'entend de tout système permettant le passage de cations métalliques et/ou d'au moins une molécule cible d'intérêt du dispositif d'extraction à travers une membrane de dialyse 18 ou de microdialyse 2 semi-perméable à l'eau et aux cations et/ou molécules ci-avant citées.

On entend par «système de microdialyse », un système de dialyse à très petite échelle. A titre d'exemple, une technique de microdialyse nécessite l'insertion d'un petit cathéter de microdialyse, également appelé sonde de microdialyse 1 dans le tissu. La sonde de microdialyse est conçue pour imiter un capillaire sanguin et se compose d'un tuyau avec une membrane semi-perméable à son extrémité, par exemple une membrane de fibres creuses, qui est reliée à la tubulure d'entrée et de sortie. La microdialyse permet de n'extraire ou de ne délivrer que les composés capables de passer à travers une membrane semi-perméable dont le seuil de coupure est choisi suivant l'application visée. Dans le cas de la dialyse, il s'agit souvent d'un phénomène dynamique de diffusion, guidé par l'écart de concentration des espèces diffusantes entre chaque côté de la membrane de dialyse.

Lors de l'utilisation de systèmes de dialyse pour extraire des composés en faibles concentrations (cations métalliques et/ou molécules cibles), la force motrice est souvent vite limitée ou saturée et le piégeage du ou des composés concernés limité par la concentration d'équilibre. Avantageusement, un système de microdialyse permet de contourner les problèmes des chélatants ou ligands classiques et d'extraire, par voie locale ou plus générale, une très forte proportion des cations métalliques ciblés (ou de cations métalliques et de molécules cibles), grâce au maintien à l'intérieur de la membrane de dialyse des espèces chimiques complexantes (agent(s) chélatant(s) et/ou ligand(s)), d'au moins un cation métallique cible et/ou une molécule cible. Les agents chélatants sont avantageusement greffés sur des macromolécules ou des nanoparticules qui possèdent une masse supérieure au seuil de coupure de la membrane afin que les espèces complexantes restent au sein du fluide de perfusion d'un côté de la membrane de dialyse. De même, les ligands sont avantageusement présents au sein ou greffés sur des macromolécules ou des nanoparticules qui possèdent, ou possèdent par nature, une masse supérieure au seuil de coupure de la membrane. Le système de dialyse contenant les espèces complexantes est placé au niveau de la zone d'intérêt, par exemple au niveau du cerveau (Figure 1) dans le cas du traitement de maladies neurodégénératives ou à proximité de la moelle épinière (Figure 2). Les cations métalliques et/ou molécules cibles étant plus petits que le seuil de coupure de la membrane de dialyse vont pouvoir diffuser à travers la membrane jusqu'au fluide de perfusion comprenant les agents chélatants et/ou ligands. Les fortes propriétés de complexation des agents chélatants et/ou ligands utilisés vont permettre la chélation des métaux cibles et/ou des molécules cibles même s'ils sont présents en très faibles quantités dans le fluide biologique. A titre d'exemple, les composés cibles (cations métalliques et/ou molécules) peuvent être présents en faible quantité dans le fluide biologique car ils sont sous forme d'agrégats biologiques. La chélation des cations métalliques et/ou la liaison avec les molécules cibles va donc diminuer la concentration des composés à extraire dans la solution à l'intérieur du réservoir comprenant le fluide de perfusion permettant de maintenir un fort gradient de concentration en composés à extraire de part et d'autre de la membrane de dialyse 18 ou de microdialyse 2, permettant ainsi de prolonger l'extraction et de maintenir un flux de composés cibles. Afin de ne pas perturber l'homéostasie des autres composés tels que les autres cations métalliques et les autres molécules présents dans le fluide biologique, ces éléments pourront être compris dans le fluide de perfusion en concentration équivalente à celle du fluide biologique.

Les dispositifs de dialyse ou microdialyse connus de l'homme du métier peuvent être utilisés, à la condition qu'ils contiennent une membrane de dialyse semi-perméable et un réservoir comprenant un fluide de perfusion contenant au moins un agent chélatant et/ou un ligand tels que mentionnés ci-dessus. A titre d'exemple, les dispositifs pouvant être utilisés sont les dispositifs médicaux développés par les sociétés M Dialysis AB, Sweden ; Integra Life Sciences ; notamment tels que les cathéters de microdialyse (références 8010509, P000049, 8010337, cette liste n'étant pas exhaustive...).

Les systèmes de dialyse, avantageusement de dialyse compacte, ou ceux de microdialyse peuvent être utilisés avec un réservoir d'échange à volume fixe 22 ou 23. La captation spécifique des cations à extraire grâce à l'agent chélatant et celle de la molécule cible grâce au ligand permettent de maintenir un gradient de purification entre les fluides biologiques à purifier et le fluide de perfusion, même pour de petits volumes de fluides sans circulation. Dans un tel mode de réalisation, les composants des liquides biologiques que l'on souhaite purifier sont préservés.

Selon un mode de réalisation, le système de dialyse comprend une membrane de dialyse 18 et un réservoir à un volume fixe 23, préférentiellement sans circulation de fluide. Avantageusement, le réservoir présente un volume inférieur à 100 ml, de préférence inférieur à 20ml et, plus préférentiellement encore inférieur à 10 ml. Un tel système de dialyse est particulièrement adapté pour un fluide biologique tel que le liquide cérébro-spinal.

Avantageusement, et selon un mode de réalisation préféré, le système de dialyse ou de microdialyse comprend une sonde de microdialyse 1 perfusée en continu avec un fluide de perfusion sous forme d'une solution aqueuse (perfusat) qui ressemble à la composition (ionique et/ou moléculaire) du fluide biologique environnant à un faible débit de moins de 1 mL/min et de préférence moins de 0,1 mL/min. Selon un autre mode de réalisation, le moyen permettant l'extraction comprend une sonde de dialyse 1 perfusée en continu avec un fluide de perfusion à un débit de moins de 10 mL/min et de préférence compris entre 1 et 5 mL/min.

Dans un mode de réalisation illustré à la figure 3, le système comprend au moins :
- un réservoir de perfusion 7 comprenant le fluide de perfusion 11 et un réservoir de recueil 8 comprenant le fluide de perfusion comprenant les composés (cations métalliques et molécules cibles) extraits 12 ;
- un cathéter bidirectionnel 3 connectant la sonde de microdialyse 1 au réservoir de perfusion 7 et au réservoir de recueil 8;
- une sonde de microdialyse 1 comprenant i) un premier lumen 4 permettant le passage du fluide de perfusion 11 vers un second lumen 5, ledit seond lumen 5 permettant d'évacuer le fluide de perfusion comprenant les compoés extraits 1é vers le réservoir de recueil 8 et ii) une membrane de microdialyse 2 entre le second lumen 5 et l'extérieur de la sonde de microdialyse 1 en contact avec le fluide biologique.

La sonde de microdialyse est préférentiellement une sonde linéaire ou concentrique. Selon un mode de réalisation illustré à la figure 3, la sonde de microdialyse est une sonde concentrique, le premier lumen 4 étant avantageusement compris à l'intérieur du second lumen 5. Un tel système de dialyse ou de microdialyse permet non seulement l'extraction de cations métalliques et de molécules cibles mais également la réalisation d'analyses quantitatives et/ou qualitatives du fluide biologique.

Dans un autre mode de réalisation illustré à la figure 4, le système comprend une sonde de microdialyse 1 ainsi qu'un cathéter bidirectionnel 3 comme dans le mode précédent. Le système comprend en outre, non pas un réservoir de perfusion et un réservoir de recueil, mais un réservoir unique, dit réservoir mixte 22. Ledit réservoir mixte 22 a pour fonction de contenir un volume donné de fluide de perfusion dont la concentration en composés extraits (cations métalliques et molécules cibles, libres et/ou liés ou/et chélatés) augmente au fur et à mesure des cycles de passage du fluide dans le dispositif. Avantageusement, le système de dialyse ou de microdialyse est muni d'un système de remplacement du fluide de perfusion dans le réservoir mixte 22. Après plusieurs cycles de passage du fluide, celui saturé en composés extraits peut alors être remplacé par un fluide de perfusion exempt de composés à extraire.

Dans un autre mode de réalisation illustré à la figure 5, le système de dialyse comprend :
- une sonde à lumens séparés 17, comprenant un second cathéter 5bis permettant l'entrée du fluide biologique dans le système (indiqué « fluide biologique non purifié 19 - figure 5) et un premier cathéter 4bis permettant la sortie du fluide biologique du système (indiqué « fluide biologique purifié 20» - figure 5) ;
- un réservoir 23 comprenant i) un fluide de perfusion 11 ; ii) un compartiment de dialyse comprenant une membrane de dialyse 18 séparant le fluide de perfusion 11 du fluide biologique 19, 20, le fluide biologique entrant dans ledit compartiment de dialyse via le second cathéter 5bis et en sortant via le premier cathéter 4bis. Le cathéter bidirectionnel 3 permet la connexion entre la sonde à lumens séparés 17 et le réservoir 23 contenu dans le boitier 6 compact de dialyse. Avantageusement, le fluide de perfusion 11 n'est pas circulant. Un dispositif permet avantageusement un remplacement ponctuel dudit fluide de perfusion 11, c'est-à-dire à intervalles de temps donnés, lorsque la concentration libre en cations métalliques et/ou en molécules cibles est à l'équilibre ou proche de l'équilibre avec celle du fluide biologique. Selon un mode de réalisation, le compartiment de dialyse est placé au sein du fluide de perfusion.

Selon un mode de réalisation avantageux compatible avec l'un quelconque des modes de réalisation précédents, les réservoirs 7/8, 22 ou 23 sont compris dans un boîtier 6 comprenant en outre au moins un des éléments suivants :
- une pompe 9 miniaturisée ;
- un système de connexion verrouillable 10 apte à permettre la circulation ou l'arrêt de la circulation de fluide dans le ou les cathéters 3, optionnellement ledit système de connexion verrouillable est apte à permettre la déconnexion du boîtier 6 et de la sonde 1 ou17 afin par exemple de pouvoir remplacer l'un des deux indépendamment ;
- au moins un capteur 16 apte à permettre de mesurer la vitesse du fluide au sein du système de dialyse ou de microdialyse, la pression à l'intérieur dudit système ou tout autre paramètre relatif au fluide, par exemple sa température, et/ou sa composition ;
- un système de commande/enregistrement 15 électronique apte à calibrer les paramètres de débit ou durée de perfusion, à permettre par exemple une action sur la ou les pompes 9, comprenant par exemple une carte électronique 14, un écran de contrôle 13 apte à afficher les paramètres mesurer au sein du système de dialyse ou de microdialyse,
- un pousse seringue 21, préférentiellement compris dans le réservoir de perfusion 7 afin de faire circuler le fluide de perfusion 11 à l'intérieur du système ;
- éventuellement un dispositif de régulation et de communication avec l'extérieur.

Selon un mode préféré, le fluide de perfusion comprend i) une solution de nanoparticules comprenant à titre de principe actif au moins un agent chélatant et ii) une solution d'au moins un ligand présentant une affinité spécifique pour la molécule cible, le diamètre moyen desdites nanoparticules et celui du ligand étant supérieurs aux pores de la membrane de dialyse 18 ou de microdialyse 2. Dans un aspect, le seuil de coupure de la membrane de dialyse 18 ou de microdialyse 2 poreuse est inférieur à la masse de l'agent chélatant, c'est-à-dire à la masse de la nanoparticule comprenant au moins un agent chélatant.

Alternativement, le fluide de perfusion comprend une solution de polymères, lesdits polymères étant greffés à au moins un principe actif qui est un agent chélatant et une solution d'au moins un ligand présentant une affinité spécifique pour la molécule cible, le diamètre moyen est supérieur aux pores de ladite membrane de dialyse ou de microdialyse. Dans cet aspect, le seuil de coupure de la membrane de dialyse 18 ou de microdialyse 2 est inférieur à la masse de l'agent chélatant, c'est-à-dire à la masse du polymère sur lequel est greffé au moins un agent chélatant.

Selon l'invention, le terme « solution » s'entend d'un mélange de liquide et de particules solides, qui restent dispersées régulièrement, les particules étant souvent suffisamment petites (microscopiques ou nanoscopiques) pour que le mélange reste stable et homogène.

Selon un mode de réalisation avantageux, le fluide de perfusion est un liquide de type « liquide céphalorachidien artificiel » comprenant des agents chélatants à base de polysiloxane et/ou de chitosane. Avantageusement, il contient de l'ordre de 1 à 10 milli-moles par litre de d'agents chélatants de type EDTA, DTPA et DOTA. Selon un mode de réalisation préféré, il peut s'agir de l'une des solutions MetAEx^{®} ou proMetAEx^{®}. commercialisées de la société Mexbrain.

Selon un mode de réalisation, ledit diamètre moyen est supérieur aux pores de ladite membrane de dialyse ou de microdialyse d'au moins 10%, 20%, 30%, 40%, 50%, 60%, 70%, 80%, 90% ou 100%.

Selon l'invention, le terme « diamètre moyen » s'entend de la moyenne harmonique des diamètres des composés, notamment des nanoparticules ou des polymères, des ligands. La distribution de taille composés est par exemple mesurée à l'aide d'un granulomètre commercial, tel qu'un granulomètre Malvern Zêta Sizer Nano-S basé sur la PCS (Photon Correlation Spectroscopy) qui se caractérise par un diamètre hydrodynamique moyen. Une méthode de mesure de ce paramètre est également décrite dans la norme ISO 13321 :1996.

Dans un mode de réalisation, la solution contient plus de 1% massique de nanoparticules ou de polymères, notamment plus 2%, 3%, 4%, 5%, 6%, 7%, 8%, 9%, et de préférence plus de 10% massique.

Selon un mode de réalisation, l'agent chélatant peut être greffé sur ledit ligand spécifique de la molécule cible. Ainsi, les agents chélatants peuvent être directement fixés, par exemple par liaisons covalences et covalentes et, plus particulièrement, par liaisons peptiques, sur les ligands.

Les nanoparticules utilisables dans le dispositif d'extraction comprennent avantageusement deux caractéristiques:
- elles sont à base de polysiloxane ou de carbone,
- elles ont un diamètre hydrodynamique moyen supérieur à 3 nm et, de préférence, inférieur à 50 nm.

Dans un mode de réalisation la nanoparticule comprend à titre de principe actif au moins un agent chélatant capable de complexer les cations métalliques, ledit agent chélatant ayant une constante de complexation log(KC1) pour au moins un desdits cations métalliques supérieure à 10 et, de préférence, supérieure ou égale à 15.

Selon l'invention, le terme « nanoparticules à base de silice », s'entend des nanoparticules caractérisées par un pourcentage massique en silicium d'au moins 8%.

Selon l'invention, le terme « nanoparticules à base de polysiloxane », s'entend des nanoparticules caractérisées par un pourcentage massique en silicium d'au moins 8%.

Selon l'invention, le terme « polysiloxane », s'entend d'un polymère réticulé inorganique consistant en un enchainement de siloxanes.

Les unités structurales du polysiloxane, identiques ou différentes, sont de formule suivante :

Si(OSi)nR4-n

dans laquelle :
- R est une molécule organique liée au silicium par une liaison covalente SiC
- n est un entier compris entre 1 et 4.

A titre d'exemple préféré, le terme « polysiloxane » englobe notamment les polymères issus de la condensation par procédé sol gel de tetraéthylorthosilicate (TEOS) et de aminopropyltriethoxysilane (APTES).

Avantageusement, ladite nanoparticule comprend ainsi :
a. des polysiloxanes, avec un rapport massique en silicium d'au moins 8% de la masse totale de la nanoparticule, de préférence entre 8% et 50% de la masse totale de la nanoparticule,
b. des agents chélatants, de préférence dans une proportion comprise entre 5 et 1000, et de préférence entre 50 et 500 par nanoparticule,
c. le cas échéant, des éléments métalliques, par exemple dans une proportion comprise entre 50 et 500, et de préférence entre 100 et 200 par nanoparticule, lesdits éléments métalliques étant complexés aux agents chélatants.

Dans un mode de réalisation, les nanoparticules utilisables selon la présente invention ne comprennent pas d'éléments métalliques. En d'autres termes, dans la définition ci-dessus, ladite nanoparticule comprend uniquement les éléments a. (des polysiloxanes ou le silicium) et b. (les agents chélatants).

Dans un mode de réalisation, les agents chélatants complexent les cations des métaux Cu, Fe, Zn, Hg, Cd, Pb, Mn, Al, Ca, Mg, Gd.

Dans un mode de réalisation, les agents chélatants sont obtenus par greffage (liaison covalente) sur la nanoparticule de l'une des molécules complexantes suivantes ou ses dérivés, telles que les acides polycarboxyliques polyaminés et leurs dérivés, notamment choisis parmi : DOTA (acide 1,4,7,10-tétraazacyclododécane-N,N',N",N"'-tétraacétique), DTPA (acide diéthylène triamine penta-acétique), DO3A-pyridine de formule (I) ci-dessous: 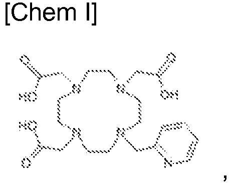 EDTA (acide 2,2',2",2"'-(ethane-1,2-diyldinitrilo)tétraacétique), EGTA (acid éthylène glycol-bis(2-aminoéthyléther)-N,N,N' ,N' -tétraacetique), BAPTA (acide 1,2-bis(o-aminophénoxy)éthane-N,N,N' ,N' -tétraacetique), NOTA (acide 1,4,7-triazacyclononane-1,4,7-triacétique), DOTAGA ((acide 2-(4,7,10-tris(carboxymethyl)-1,4,7,10-tétraazacyclododecan-1-yl)pentanedioic), DFO (deferoxamine), les dérivés amides comme par exemple le DOTAM (1,4,7,10-tetrakis(carbamoylmethyl)-1,4,7,10 tetraazacyclododecane) ou le NOTAM (1,4,7-tetrakis(carbamoylmethyl)-1,4,7-triazacyclononane), ainsi que les dérivés mixtes acides carboxiliques/amides, les dérivés phosphoniques comme par exemple le DOTP (1,4,7,10- tetraazacyclododecane1,4,7,10-tetrakis(methylene phosphonate)) ou le NOTP (1,4,7-tetrakis(methylene phosphonate)-1,4,7-triazacyclononane), les dérivés du cyclame comme TETA (1,4,8,11-tetraazacyclotetradecane-N,N',N",N"'- tetraacetic acid), TETAM (1,4,8,11-tetraazacyclotetradecane-N,N',N",N"'- tetrakis(carbamoylmethyl)), TETP (1,4,8,11-tetraazacyclotetradecane-N,N',N",N"'- tetrakis(methylene phosphonate)) ou leurs mélanges.

De préférence, lesdits agents chélatants ci-dessus sont liés directement ou indirectement par liaison covalente aux siliciums des polysiloxanes de la nanoparticule. Le terme liaison « indirecte », s'entend de la présence d'un « linker » moléculaire ou « espaceur » entre la nanoparticule et l'agent chélatant, ledit linker ou espaceur étant lié de manière covalente à l'un des constituants de la nanoparticule.

Selon un mode de réalisation préféré, ladite nanoparticule est une nanoparticule à base de polysiloxane d'un diamètre hydrodynamique moyen compris entre 3 et 100 nm, comprenant l'agent chélatant obtenu par greffage de DOTA, DOTAGA, EDTA ou du DTPA sur la nanoparticule.

Selon un mode de réalisation préféré, ladite nanoparticule est une nanoparticule de diamètre moyen supérieur à 20 kDa et inférieur à 1 MDa, comprenant l'agent chélatant obtenu par greffage de DOTA, DOTAGA, EDTA ou du DTPA sur la nanoparticule.

Selon un mode de réalisation préféré, ladite solution comprenant lesdites nanoparticules, contient également des oligoéléments, choisis parmi Calcium, Magnésium, Fer, Cuivre, Zinc ou Manganèse.

Dans un autre mode de réalisation de l'invention, des polymères peuvent être utilisés à la place des nanoparticules susmentionnées. Dans un tel cas, lesdits polymères sont greffés à au moins un agent chélatant.

On entend par « polymère » toute macromolécule formée de l'enchaînement covalent d'un très grand nombre d'unités de répétition qui dérivent d'un ou de plusieurs monomères. Les polymères préférentiellement utilisés sont par exemple de la famille des chitosanes, des polyacrylamides, polyamines ou polycarboxyliques, polyéthylèneglycols, polyvinylalcools (PVA). Par exemple, il peut s'agit de polymères contenant des fonctions aminées tels que le chitosane. Selon un mode de réalisation préféré, ledit polymère est biocompatible.

Dans un mode de réalisation, les agents chélatants ou leurs dérivés greffés sur lesdits polymères sont des acides polycarboxyliques polyaminés et leurs dérivés, notamment choisis parmi : DOTA, DTPA, DO3A-pyridine de formule (I) ci-dessus, EDTA, EGTA, BAPTA, NOTA, DOTAGA, DFO, DOTAM, NOTAM, DOTP, NOTP, TETA, TETAM et TETP ou leurs mélanges.

De préférence, lesdits agents chélatants ci-dessus sont liés directement ou indirectement par liaison covalente au polymère ou à une chaine de polymères de plus de 10 kDa et de préférence de plus de 100 kDa. Le terme liaison « indirecte », s'entend de la présence d'un « linker » moléculaire ou « espaceur » entre le polymère et l'agent chélatant, ledit linker ou espaceur étant lié de manière covalente à l'un des constituants dudit polymère.

Dans un mode de réalisation, les agents chélatants ou leurs dérivés greffés sur lesdits polymères comprendront des fonctions dithiocarbamates.

Selon un mode de réalisation préféré, ledit polymère greffé à un agent chélatant est choisi parmi : le chitosane greffé à du DPTA-BA ou le chitosane greffé à du DFO ou le chitosane greffé à du EDTA-BA ou le chitosane greffé à du DOTAGA-A.

Selon un mode de réalisation préféré, ladite solution comprenant lesdits polymères contient également des oligoéléments, choisis parmi Calcium, Magnésium, Fer, Cuivre, Zinc, ou Manganèse.

Alternativement, le fluide de perfusion est une solution de molécules chélatantes. Lesdites molécules chélatantes peuvent avoir un diamètre moyen supérieur aux pores de ladite membrane de dialyse ou de microdialyse, c'est-à-dire supérieur au seuil de coupure de la membrane afin d'être maintenues au sein du liquide de la membrane de dialyse. Dans un autre mode de réalisation, elles peuvent avoir un diamètre moyen inférieur aux pores de ladite membrane de dialyse ou de microdialyse et, dans ce cas, elles peuvent passer à travers les pores de la membrane avant de passer dans l'organisme et être naturellement éliminées de façon naturelle par les reins ou le foie.

### Application industrielle

L'invention peut trouver à s'appliquer notamment dans le maintien de l'homéostasie, en particulier le maintien de l'homéostasie de deux composés cibles tels qu'un cation métallique et une molécule cible.

Selon un mode de réalisation préféré, le dispositif d'extraction conjointe d'au moins un cation métallique et d'au moins une molécule cible d'un fluide biologique ou d'un agrégat biologique susmentionné est utilisé dans le traitement d'une maladie choisie parmi :
- des amyloses systémiques et/ou localisées ; notamment des amyloses choisies parmi : amylose de type AL (chaines légères d'immunoglobulines), amylose de type AH (chaines lourdes d'immunoglobulines, amylose AA (protéine amyloïde sérique), amylose ATTR (transthyrétine), cardiopathies amyloïdes, amyloses rénales, diabète de type II, maladies à Prion ou maladies liées à une protéine amyloïde. Les amyloses sont aussi impliquées dans les maladies neurodégénératives, notamment la maladie d'Alzheimer (amyloïde-beta), la maladie de Parkinson (alpha synucléine), la sclérose latérale amyotrophie (superoxyde dismutase), la maladie de Huntington (Huntingtin.
- des tauopathies notamment :des tauopathies choisies parmi : maladie d'Alzheimer, paralysie supranucléaire progressive, démence fronto-temporale ;
- des pathologies présentant un dépôt à base d'au moins une protéine;
- des maladies présentant une dyshoméostasie métallique notamment la maladie de Wilson ou des troubles neurologiques sans caractéristique amyloïde, tels que l'autisme ou la schizophrénie, ou troubles neurologiques avec caractéristique amyloïde tels les amyloses citées ci-dessus touchant ou non le système nerveux central et/ou périphérique.

Selon un mode de réalisation préféré, le dispositif d'extraction conjointe d'au moins un cation métallique et d'au moins une molécule cible d'un agrégat biologique susmentionné est utilisé pour ralentir la formation, dissocier ou dissoudre un agrégat biologique, préférentiellement sous forme d'oligomères, de fibrilles ou de plaques comprenant au moins la molécule cible. est utilisé dans le diagnostic, la prévention et/ou la thérapie.

Un présent aspect de la demande mais ne faisant pas partie de l'invention, concerne également une méthode pour extraire des cations métalliques et molécules cibles chez un sujet comprenant l'administration d'un implant sur lequel est greffé au moins un agent chélatant, ou bien l'utilisation d'un fluide de perfusion contenant au moins un agent chélatant au sein d'un dispositif tel que ceux susmentionnés.

Selon l'invention, ledit « sujet » s'entend d'un homme ou d'un animal à prévenir ou à traiter.

L'invention ne se limite pas à la description précédente mais elle englobe toutes les variantes que pourra envisager l'homme de l'art dans le cadre de la protection recherchée; l'étendue de la protection recherchée par la présente invention est limitée par le jeu des revendications.

### Liste des signes de référence

1 . Sonde de microdialyse
2 . Membrane de microdialyse
3 . Cathéter bidirectionnel
4 . Premier lumen
4bis . Premier cathéter
5 . Second lumen
5bis . Second cathéter
6 . Boîtier compact
7. Réservoir de perfusion
8. Réservoir de recueil
9. Pompe
10 . Système de connexion verrouillable
11 . Fluide de perfusion
12 . Fluide de perfusion comprenant les composés (cations métalliques et molécules cibles) extraits
13 . Ecran de contrôle (affichage des paramètres)
14 . Carte électronique
15 . Commande des paramètres
16 . Capteurs
17 . Sonde double lumen
18 . Membrane de dialyse
19 . Fluide biologique non purifié
20 . Fluide biologique purifié
21 : Pousse seringue
22 : Réservoir mixte
23 : Réservoir

### Liste des documents cités

### Documents brevets

À toute fin utile, le(s) document(s) brevet(s) suivant(s) est (sont) cité(s) :
- US2013/0045216 A1 (Appl. No. : US13/655,234)

### Littérature non-brevet

À toute fin utile, , le(s) élément(s) non-brevet(s) suivant(s) est (sont) cité(s) :
- G. Crisponi, V. M. Nurchi, V. Bertolasi, M. Remelli, G. Faa, « Chelating agents for human diseases related to aluminium overload », Coordination Chemistry Reviews, Volume 256, Issues 1-2, January 2012, pp. 89-104
- E. J. McAllum et D.Finkelstein, « Metals in Alzheimer's and Parkinson's Disease: Relevance to Dementia with Lewy Bodies", Journal of Molecular Neuroscience, Volume 60(3), Août 2016, pp. 279-288]
- S. Wiethoff et H. Houlden "Neurodegeneration with brain iron accumulation", Handb. Clin. Neurol., Volume 145, 2017, pp. 57-166
- J Acosta-Cabronero, Matthew J. Betts, Arturo Cardenas-Blanco, Shan Yang et Peter J. Nestor, "In Vivo MRI Mapping of Brain Iron Deposition across the Adult Lifespan", Journal of Neuroscience, Janvier 2016, 36 (2) pp. 364-374;
- A. A. Belaidi et A. I. Bush, "Iron neurochemistry in Alzheimer's disease and Parkinson's disease: targets for therapeutics", Journal of Neurochemistry, Vol. 139, Issue S1, 2016, pp. 179-197
- Simon J.A. van den Bogaard, Eve M. Dumas, Raymund A.C. Roos "Metal Related Neurodegenerative Disease - International Review of Neurobiology, Volume 110, 2013, pp. 241-250 https://www.sciencedirect.com/science/article/pii/B9780124105027000119,
- G. Bartzokis, J. Cummings, S. Perlman, D. B. Hance, J. Mintz, Increased basal ganglia iron levels in Huntington disease, Arch. Neurol., 1999, 5, 569-574. https://jamanetwork.com/journals/jamaneurology/fullarticle/775016
- M. Meendez-Gonzalez, H. S. Padilla-Zambrano, G. Alvarez, E. Capetillo-Zarate, C. Tomas-Zapico, A. Costa, "Targeting beta-amyloid at the CSF: a new therapeutic strategy in Alzheimer's didease", Hypothesis and Theory, Vol. 10 (100), Avril 2018; DOI: 10.3389/fnagi.2018.00100
- Menéndez González Mechanical, "Dilution of Beta-amyloid Peptide and Phosphorylated Tau Protein in Alzheimer's Disease: Too Simple to be True?", Cureus. 2017 Feb 28;9(2):e1062. doi: 10.7759/cureus.1062.
- Tõugu V., Tiiman A, Palumaa P, "Interactions of Zn(II) and Cu(II) ions with Alzheimer's amyloid-beta peptide. Metal ion binding, contribution to fibrillization and toxicity.", Metallomics. 2011 Mar;3(3):250-61. doi: 10.1039/c0mt00073f.
- Cremades N, Dobson CM, "The contribution of biophysical and structural studies of protein self-assembly to the design of therapeutic strategies for amyloid diseases", Neurobiol Dis. 2018 Jan;109 (Pt B):178-190. doi: 10.1016/j.nbd.2017.07.009.
- A Czlonkowska, T. Litwin, P. Dusek, P. Ferenci, S. Lutsenko, V. Medici, J. K. Rybakowski, K. H. Weiss, M. L. Schilsky, Wilson disease, Nature Rev. Dis. Primers, 2018, 4:21. https://www.nature.com/articles/s41572-018-0018-3.
- Pfaender S, Grabrucker AM. Characterization of biometal profiles in neurological disorders. Metallomics. 2014 May;6(5):960-77.
- Boland B, Yu WH, Corti O, Mollereau B, Henriques A, Bezard E, Pastores GM, Rubinsztein DC, Nixon RA, Duchen MR, Mallucci GR, Kroemer G, Levine B, Eskelinen EL, Mochel F, Spedding M, Louis C, Martin OR, Millan MJ. Promoting the clearance of neurotoxic proteins in neurodegenerative disorders of ageing. Nat Rev Drug Discov. 2018 Sep;17(9):660-688.
- ladanza MG, Jackson MP, Hewitt EW, Ranson NA, Radford SE.A new era for understanding amyloid structures and disease. Nat Rev Mol Cell Biol. 2018 Dec;19(12):755-773.

## Revendications

1. Dispositif d'extraction conjointe d'au moins un cation métallique et d'au moins une molécule cible d'un fluide biologique d'un patientà des fins thérapeutiques, **caractérisé en ce qu'**il comprend un système de dialyse ou de microdialyse comprenant une membrane de dialyse et un réservoir comprenant un fluide de perfusion, ledit fluide de perfusion étant choisi parmi :
- une solution de nanoparticules, lesdites nanoparticules étant greffées à au moins un principe actif qui est un agent chélatant dudit cation métallique et une solution d'au moins un ligand présentant une affinité spécifique pour la molécule cible, le diamètre moyen desdites nanoparticules et celui du ligand étant supérieurs aux pores de la membrane de dialyse,
- une solution de polymères, lesdits polymères étant greffés à au moins un principe actif qui est un agent chélatant dudit cation métallique et une solution d'au moins un ligand présentant une affinité spécifique pour la molécule cible, le diamètre moyen desdits polymères et dudit ligand étant supérieur aux pores de ladite membrane de dialyse.

2. Dispositif selon la revendication 1 **caractérisé en ce que** :
- la constante de complexation log(KC1) de l'agent chélatant pour au moins un cation métallique est supérieure à 10, de préférence, supérieure ou égale à 15 et
- ledit au moins un cation est choisi parmi les cations des métaux Cu, Fe, Zn, Hg, Cd, Pb, Mn, Co, Gd et Al, pris seul ou en association et plus particulièrement Cu, Fe et Zn, pris seul ou en association.

3. Dispositif selon l'une quelconque des revendications précédentes **caractérisé en ce que** les agents chélatants sont obtenus par greffage sur les nanoparticules ou sur le polymère de l'une des molécules complexantes suivantes: DOTA, DTPA, EDTA, EGTA, BAPTA, NOTA, DOTAGA, DFO, DOTAM, NOTAM, DOTP, NOTP, TETA, TETAM, TETP et DTPABA, ou leurs mélanges.

4. Dispositif selon l'une quelconque des revendications précédentes **caractérisé en ce que** lesdites nanoparticules sont des nanoparticules à base de polysiloxane d'un diamètre moyen compris entre 3 et 50 nm, comprenant l'agent chélatant obtenu par greffage de DOTA, DOTAGA, EDTA ou DTPA sur les nanoparticules.

5. Dispositif selon l'une quelconque des revendications précédentes **caractérisé en ce que** l'agent chélatant contient au moins un cation alcalino-terreux, préférentiellement un cation de métaux choisis parmi Ca et Mg.

6. Dispositif selon l'une quelconque des revendications précédentes **caractérisé en ce que** lesdites nanoparticules sont à base de polysiloxane ou lesdits polymères sont à base de chitosane ou de polyéthylèneglycol ou de polyvinylalcools.

7. Dispositif selon l'une quelconque des revendications précédentes **caractérisé en ce que** la molécule cible est choisie parmi les protéines, les peptides, et les glycoprotéines,
en particulier la molécule cible est choisie parmi les protéines amyloïdogéniques et des composants des structures amyloïdes,
en particulier la molécule cible est choisie parmi les protéines et/ou leurs précurseurs telles les chaînes d'immunoglobuline légères et lourdes, la protéine amyloïde sérique, la transthyrétine, l'apolipoprotéine AI, AII, AVI, CII ou CIII, la microglobuline beta 2, la gelsoline, le lysosyme, le fibrinogène, la cystatine C, le facteur atrial natriurétique, la calcitonine, l'amyline, l'insuline, la prolactine, la lactoferrine, la cadhérine, A-Bri, A-Dan, le peptide beta amyloïde, la protéine prion, l'alpha-synucléine, la protéine Tau, la superoxydismutase, la huntingtin, la neuroserpine, l'actine, la ferritine ou leurs mélanges.

8. Dispositif selon l'une quelconque des revendications précédentes **caractérisé en ce que** la molécule cible est choisie parmi les molécules impliquées dans les amyloses, les tauopathies ou toute pathologie présentant un dépôt à base d'une ou plusieurs protéines.

9. Dispositif selon l'une quelconque des revendications précédentes **caractérisé en ce que** le ligand est un anticorps ou un ligand protéique artificiel de la molécule cible.

10. Dispositif selon l'une quelconque des revendications précédentes **caractérisé en ce que** le ligand est choisi parmi:
- les anticorps ciblant la protéine béta-amyloide, préférentiellement Solanezumab, Aducanumab, Crenezumab, Ponezumab, GSK933776, Gantenerumab, AAB-003, AAB-001, BAN2401, LY2599666, LY3002813, LY3372993, MEDI1814, SAR228810 ;
- les anticorps ciblant l'alpha-synucléine, préférentiellement BII054 ou PRX002 ;
- les anticorps ciblant la protéine tau, préférentiellement BII076, BII092, ABBV-8E12, JNJ-63733657, LY3303560, RG7345, RO7105705, UCB0107;
- les anticorps ciblant la protéine amyloïde sérique, préférentiellement : Dezamizumab ou GSK2398852, Miridesap ou GSK2315698, GSK3039294 ;
- les anticorps ciblant la transthyrétine, préférentiellement PRX004 ;
- les aptamères ;
- les ligands protéiques artificiels présentant une affinité spécifique pour l'une au moins des molécules, optionnellement choisis parmi : ABD, Adhiron, Adnectin, Affibody, Affilin, Affimer, Affitin, Alphabody, Anticalin, Armadillo repeat proteins, Atrimer/tetranectin, Avimer/Maxibody, Centyrin, DARPin1 ;
- les ligands protéiques artificiels de moins de 50 kDA, de préférence moins de 30 kDa et, plus préférentiellement de moins de 5kDa, greffées sur une nanoparticules ou un polymère de plus de 5 nm de diamètre hydrodynamique, préférentiellement de plus de 100 kDa, ou
- leurs mélanges.

11. Dispositif selon l'une quelconque des revendications précédentes pour son utilisation chez un patient présentant une pathologie parmi :
- les amyloses systémiques et/ou localisées ; préférentiellement les amyloses choisies parmi : amylose de type AL (chaines légères d'immunoglobulines), amylose de type AH (chaines lourdes d'immunoglobulines, amylose AA (protéine amyloïde sérique), amylose ATTR (transthyrétine), cardiopathies amyloïdes, amyloses rénales, diabète de type II, maladies à Prion ou maladies liées à une protéine amyloïde ;
- les tauopathies préférentiellement choisies parmi : maladie d'Alzheimer, paralysie supranucléaire progressive, démence fronto-temporale ;
- les pathologies présentant un dépôt à base d'au moins une protéique;
- les maladies présentant une dyshoméostasie métallique préférentiellement la maladie de Wilson ou des troubles neurologiques sans caractéristique amyloïde tels que l' autisme ou la schizophrénie, ...), ou les troubles neurologiques avec caractéristique amyloïde tels que les amyloses touchant ou non le système nerveux central et/ou périphérique.

12. Système de microdialyse comprenant un dispositif d'extraction selon l'une quelconque des revendications précédentes et tel qu'il comprend au moins:
- un réservoir de perfusion (7) comprenant le fluide de perfusion (11) et un réservoir de recueil (8) comprenant le fluide de perfusion comprenant les composés extraits (12); ou un réservoir mixte (22) comprenant le fluide de perfusion (11, 12) ;
- un cathéter bidirectionnel (3) connectant la sonde de microdialyse (1) au réservoir de perfusion (7) et au réservoir de recueil (8) ou au réservoir mixte (22) ;
- une sonde de microdialyse (1) comprenant un premier lumen (4) permettant le passage du fluide de perfusion (11) vers un second lumen (5), ledit second lumen (5) permettant d'évacuer le fluide de perfusion comprenant les composés extraits (12) et une membrane de microdialyse (2) comprise entre le second lumen (5) et l'extérieur de la sonde de microdialyse (1) en contact avec le fluide biologique.

13. Système de dialyse comprenant un dispositif d'extraction selon l'une quelconque des revendications précédentes et tel qu'il comprend au moins :
- une sonde à lumens séparés (17), comprenant un second cathéter (5bis) permettant l'entrée du fluide biologique dans le système de dialyse et un premier cathéter (4bis) permettant la sortie du fluide biologique du système de dialyse;
- un réservoir (23) comprenant i) le fluide de perfusion (11); ii) un compartiment de dialyse comprenant une membrane de dialyse (18) séparant le fluide de perfusion (11) du fluide biologique, le fluide biologique entrant dans ledit compartiment de dialyse via le second cathéter (5bis) et en sortant via le premier cathéter (4bis).

## Patentansprüche

1. Vorrichtung zur gemeinsamen Extraktion wenigstens eines Metallkations und wenigstens eines Zielmoleküls aus einer biologischen Flüssigkeit eines Patienten zu therapeutischen Zwecken, **dadurch gekennzeichnet, dass** sie ein Dialyse- oder Mikrodialysesystem umfasst, welches eine Dialysemembran und einen Behälter mit einer Perfusionsflüssigkeit umfasst, wobei die Perfusionsflüssigkeit ausgewählt ist, aus:
- einer Lösung von Nanopartikeln, wobei die Nanopartikel mit wenigstens einem Wirkstoff gepfropft sind, welcher ein Chelatbildner für das Metallkation ist, und einer Lösung von wenigstens einem Liganden, welcher eine spezifische Affinität für das Zielmolekül aufweist, wobei der durchschnittliche Durchmesser der Nanopartikel und der des Liganden größer ist als die Poren der Dialysemembran,
- einer Polymerlösung, wobei die Polymere mit wenigstens einem Wirkstoff gepfropft sind, welcher ein Chelatbildner für das Metallkation ist, und einer Lösung von wenigstens einem Liganden, welcher eine spezifische Affinität für das Zielmolekül aufweist, wobei der mittlere Durchmesser der Polymere und des Liganden größer ist als die Poren der Dialysemembran.

2. Vorrichtung nach Anspruch 1, **dadurch gekennzeichnet, dass**:
- die Komplexierungskonstante log(KC1 ) des Chelatbildners für wenigstens ein Metallkation größer als 10, vorzugsweise größer oder gleich 15 ist, und
- das wenigstens eine Kation aus den Kationen der Metalle Cu, Fe, Zn, Hg, Cd, Pb, Mn, Co, Gd und Al, einzeln oder in Kombination, und insbesondere Cu, Fe und Zn, einzeln oder in Kombination, ausgewählt ist.

3. Vorrichtung nach einem der vorstehenden Ansprüche, **dadurch gekennzeichnet, dass** die Chelatbildner durch Aufpfropfen eines der folgenden komplexbildenden Moleküle auf die Nanopartikel oder das Polymer erhalten werden: DOTA, DTPA, EDTA, EGTA, BAPTA, NOTA, DOTAGA, DFO, DOTAM, NOTAM, DOTP, NOTP, TETA, TETAM, TETP und DTPABA oder deren Mischungen.

4. Vorrichtung nach einem der vorstehenden Ansprüche, **dadurch gekennzeichnet, dass** die Nanopartikel Nanopartikel auf Polysiloxanbasis mit einem durchschnittlichen Durchmesser zwischen 3 und 50 nm sind, welche den Chelatbildner umfassen, welcher durch Pfropfen von DOTA, DOTAGA, EDTA oder DTPA auf die Nanopartikel erhalten wird.

5. Vorrichtung nach einem der vorstehenden Ansprüche, **dadurch gekennzeichnet, dass** der Chelatbildner wenigstens ein Erdalkalimetallkation enthält, vorzugsweise ein Metallkation, welches aus Ca und Mg ausgewählt ist.

6. Vorrichtung nach einem der vorstehenden Ansprüche, **dadurch gekennzeichnet, dass** die Nanopartikel auf Polysiloxan basieren oder die Polymere auf Chitosan oder Polyethylenglykol oder Polyvinylalkoholen basieren.

7. Vorrichtung nach einem der vorstehenden Ansprüche, **dadurch gekennzeichnet, dass** das Zielmolekül aus Proteinen, Peptiden und Glykoproteinen ausgewählt ist, insbesondere dass das Zielmolekül aus amyloidogenen Proteinen und Komponenten von Amyloidstrukturen ausgewählt ist, insbesondere ist das Zielmolekül ausgewählt aus Proteinen und/oder deren Vorläufern wie leichten und schweren Immunglobulinketten, Serumamyloidprotein, Transthyretin, Apolipoprotein AI, AII, AVI, CII oder CIII, Beta-2-Mikroglobulin, Gelsolin, Lysozym, Fibrinogen, Cystatin C, atrialer natriuretischer Faktor, Calcitonin, Amylin, Insulin, Prolaktin, Lactoferrin, Cadherin, A-Bri, A-Dan, Beta-Amyloid-Peptid, Prionprotein, Alpha-Synuclein, Tau-Protein, Superoxiddismutase, Huntingtin, Neuroserpin, Aktin, Ferritin oder deren Mischungen.

8. Vorrichtung nach einem der vorstehenden Ansprüche, **dadurch gekennzeichnet, dass** das Zielmolekül aus Molekülen ausgewählt ist, welche an Amyloidosen, Tauopathien oder jeder Pathologie beteiligt sind, welche eine Ablagerung auf der Basis eines oder mehrerer Proteine aufweist.

9. Vorrichtung nach einem der vorstehenden Ansprüche, **dadurch gekennzeichnet, dass** der Ligand ein Antikörper oder ein künstlicher Proteinligand des Zielmoleküls ist.

10. Vorrichtung nach einem der vorstehenden Ansprüche, **dadurch gekennzeichnet, dass** der Ligand ausgewählt ist, aus:
- Antikörpern, welche gegen das Beta-Amyloid-Protein gerichtet sind, vorzugsweise Solanezumab, Aducanumab, Crenezumab, Ponezumab, GSK933776, Gantenerumab, AAB-003, AAB-001, BAN2401, LY2599666, LY3002813, LY3372993, MEDI1814, SAR228810;
- Antikörper gegen Alpha-Synuclein, vorzugsweise BII054 oder PRX002;
- Antikörper gegen das Tau-Protein, vorzugsweise BII076, BII092, ABBV-8E12, JNJ-63733657, LY3303560, RG7345, RO7105705, UCB0107;
- Antikörper, welche gegen das Serum-Amyloid-Protein gerichtet sind, vorzugsweise: Dezamizumab oder GSK2398852, Miridesap oder GSK2315698, GSK3039294,
- Antikörper, welche gegen Transthyretin gerichtet sind, vorzugsweise PRX004;
- Aptamere;
- künstliche Proteinliganden mit einer spezifischen Affinität für wenigstens eines der Moleküle, wahlweise ausgewählt aus: ABD, Adhiron, Adnectin, Affibody, Affilin, Affimer, Affitin, Alphabody, Anticalin, Armadillo-Repeat-Proteine, Atrimer/Tetranectin, Avimer/Maxibody, Centyrin, DARPin1;
- künstliche Proteinliganden mit weniger als 50 kDA, vorzugsweise weniger als 30 kDa und noch bevorzugter weniger als 5 kDa, gepfropft auf Nanopartikel oder ein Polymer mit einem hydrodynamischen Durchmesser von mehr als 5 nm, vorzugsweise mehr als 100 kDa, oder
- deren Mischungen.

11. Vorrichtung nach einem der vorstehenden Ansprüche zur Verwendung bei einem Patienten mit einer der folgenden Erkrankungen:
- systemische und/oder lokalisierte Amyloidosen; vorzugsweise Amyloidosen ausgewählt aus: Amyloidose vom Typ AL (leichte Ketten von Immunglobulinen), Amyloidose vom Typ AH (schwere Ketten von Immunglobulinen), Amyloidose AA (Serum-Amyloidprotein), Amyloidose ATTR (Transthyretin), Amyloid-Kardiopathien, renale Amyloidosen, Typ-II-Diabetes, Prionenerkrankungen oder Erkrankungen im Zusammenhang mit einem Amyloidprotein;
- Tauopathien, vorzugsweise ausgewählt aus: Alzheimer-Krankheit, progressive supranukleäre Lähmung, frontotemporale Demenz;
- Erkrankungen mit Ablagerungen auf Basis wenigstens eines Proteins;
- Erkrankungen mit Metallhomöostase, vorzugsweise Morbus Wilson oder neurologische Störungen ohne Amyloid-Merkmale wie Autismus oder Schizophrenie, ...), oder neurologische Störungen mit Amyloid-Merkmalen wie Amyloidosen, welche das zentrale und/oder periphere Nervensystem betreffen oder nicht.

12. Mikrodialysesystem, umfassend eine Vorrichtung zur Extraktion nach einem der vorstehenden Ansprüche, wobei es wenigstens umfasst:
- einen Perfusionsbehälter (7), welcher die Perfusionsflüssigkeit (11) umfasst, und einen Auffangbehälter (8), welcher die Perfusionsflüssigkeit umfasst, welche die extrahierten Verbindungen (12) enthält; oder einen gemischten Behälter (22), welcher die Perfusionsflüssigkeit (11, 12) umfasst,
- einen bidirektionalen Katheter (3), welcher die Mikrodialysesonde (1) mit dem Perfusionsbehälter (7) und dem Auffangbehälter (8) oder dem Mischbehälter (22) verbindet;
- eine Mikrodialysesonde (1), welche ein erstes Lumen (4), welches den Durchfluss der Perfusionsflüssigkeit (11) zu einem zweiten Lumen (5) ermöglicht, wobei das zweite Lumen (5) den Abfluss der Perfusionsflüssigkeit mit den extrahierten Verbindungen (12) ermöglicht, und eine Mikrodialysemembran (2) zwischen dem zweiten Lumen (5) und der Außenseite der Mikrodialysesonde (1) in Kontakt mit der biologischen Flüssigkeit.

13. Dialysesystem, umfassend eine Vorrichtung zur Extraktion nach einem der vorstehenden Ansprüche, welches wenigstens umfasst:
- eine Sonde mit getrennten Lumen (17), welche einen zweiten Katheter (5bis), welcher den Eintritt der biologischen Flüssigkeit in das Dialysesystem ermöglicht, und einen ersten Katheter (4bis) umfasst, welcher den Austritt der biologischen Flüssigkeit aus dem Dialysesystem ermöglicht;
- einen Behälter (23), umfassend i) die Perfusionsflüssigkeit (11); ii) eine Dialysekammer mit einer Dialysemembran (18), welche die Perfusionsflüssigkeit (11) von der biologischen Flüssigkeit trennt, wobei die biologische Flüssigkeit über den zweiten Katheter (5bis) in die Dialysekammer eintritt und über den ersten Katheter (4bis) aus dieser austritt.

## Claims

1. A device for the simultaneous extraction of at least one metal cation and at least one target molecule from a patient's biological fluid for therapeutic purposes, **characterised in that** it includes a dialysis or microdialysis system comprising a dialysis membrane and a reservoir comprising a perfusion fluid, said perfusion fluid being selected from:
- a solution of nanoparticles, with said nanoparticles being grafted to at least one active ingredient that is a chelating agent for the metal cation, and a solution of at least one ligand having a specific affinity for the target molecule, the mean diameter of said nanoparticles and that of the ligand being greater than the pores of the dialysis membrane,
- a solution of polymers, with said polymers being grafted to at least one active ingredient that is a chelating agent for the metal cation, and a solution of at least one ligand having a specific affinity for the target molecule, the mean diameter of said polymers and said ligand being greater than the pores of the dialysis membrane.

2. The device according to claim 1, **characterised in that**:
- the complexation constant log(KC1) of the chelating agent for at least one metal cation is greater than 10, preferably greater than or equal to 15 and
- said at least one cation is selected from cations of the metals Cu, Fe, Zn, Hg, Cd, Pb, Mn, Co, Gd, and Al, either alone or in combination, and more particularly Cu, Fe, and Zn, either alone or in combination.

3. The device according to any one of the preceding claims, **characterised in that** the chelating agents are obtained by grafting, onto the nanoparticles or onto the polymer, one of the following complexing molecules:
DOTA, DTPA, EDTA, EGTA, BAPTA, NOTE, DOTAGA, DFO, DOTAM, NOTAM, DOTP, NOTP, TETA, TETAM, TETP and DTPABA, or mixtures thereof.

4. The device according to any one of the preceding claims, **characterised in that** said nanoparticles are polysiloxane-based nanoparticles with a mean diameter between 3 and 50 nm, comprising the chelating agent obtained by grafting DOTA, DOTAGA, EDTA or DTPA onto the nanoparticles.

5. The device according to any one of the preceding claims, **characterised in that** the chelating agent contains at least one alkaline earth cation, preferably a cation of metals selected from Ca and Mg.

6. The device according to any one of the preceding claims, **characterised in that** the nanoparticles are based on polysiloxane, or said polymers are based on chitosan, polyethylene glycol, or polyvinyl alcohols.

7. The device according to any one of the preceding claims, **characterised in that** the target molecule is selected from proteins, peptides, and glycoproteins,
in particular, the target molecule is selected from amyloidogenic proteins and components of amyloid structures,
in particular the target molecule is selected from proteins and/or precursors thereof such as light and heavy immunoglobulin chains, serum amyloid protein, transthyretin, apolipoprotein AI, AII, AVI, CII or CIII, beta-2-microglobulin, gelsoline, lysosyme, fibrinogen, cystatin C, natriuretic atrial factor, calcitonin, amyline, insulin, prolactin, lactoferrin, cadherin, A-Bri, A-Dan, amyloid beta peptide, prion protein, alpha-synuclein, Tau protein, superoxydismutase, huntingtin, neuroserpine, actin, ferritin, or mixtures thereof.

8. The device according to any one of the preceding claims, **characterised in that** the target molecule is selected from molecules involved in amyloidoses, tauopathies, or any pathology having a deposition based on one or more proteins.

9. The device according to any one of the preceding claims, **characterised in that** the ligand is either an antibody or an artificial protein ligand of the target molecule.

10. The device according to any one of the preceding claims, **characterised in that** the ligand is selected from:
- antibodies targeting the beta-amyloid protein, preferably Solanezumab, Aducanumab, Crenezumab, Ponezumab, GSK933776, Gantenerumab, AAB-003, AAB-001, BAN2401, LY2599666, LY3002813, LY3372993, MEDI1814, SAR228810;
- antibodies targeting alpha-synuclein, preferably BII054 or PRX002;
- antibodies targeting the tau protein, preferably BII076, BII092, ABBV-8E12, JNJ-63733657, LY3303560, RG7345, RO7105705, UCB0107;
- antibodies targeting the serum amyloid protein, preferably: Dezamizumab or GSK2398852, Miridesap or GSK2315698, GSK3039294;
- antibodies targeting transthyretin, preferably PRX004;
- aptamers;
- artificial protein ligands with a specific affinity for at least one of the molecules, optionally selected from: ABD, Adhiron, Adnectin, Affibody, Affilin, Affimer, Affitin, Alphabody, Anticalin, Armadillo repeat proteins, Atrimer/tetranectin, Avimer/Maxibody, Centyrin, DARPin1;
- artificial protein ligands of less than 50 kDa, preferably less than 30 kDa, and more preferably less than 5 kDa, grafted onto a nanoparticle or polymer with a hydrodynamic diameter of more than 5 nm, preferably of more than 100 kDa, or
- mixtures thereof.

11. The device according to any one of the preceding claims, for use in a patient with a pathology from:
- systemic and/or localised amyloidoses; preferably amyloidoses selected from: AL-type amyloidosis (light immunoglobulin chains), AH-type amyloidosis (heavy immunoglobulin chains), AA- amyloidosis (serum amyloid protein), ATTR- amyloidosis (transthyretin), amyloid heart diseases, renal amyloidoses, type II diabetes, Prion diseases, or diseases related to amyloid proteins;
- tauopathies preferably selected from: Alzheimer's disease, progressive supranuclear palsy, frontotemporal dementia;
- pathologies having a deposition based on at least one protein;
- diseases having a metal dyshomeostasis, preferentially Wilson's disease, or neurological disorders without amyloid characteristics, such as autism or schizophrenia, etc.), or neurological disorders with amyloid characteristics, such as amyloidoses, which may or may not affect the central and/or peripheral nervous system.

12. A microdialysis system comprising an extraction device according to any one of the preceding claims, and such that it comprises at least:
- an infusion reservoir (7) containing the infusion fluid (11) and a collection reservoir (8) comprising the infusion fluid comprising the extracted compounds (12); or a mixed reservoir (22) comprising the infusion fluid (11, 12);
- a bidirectional catheter (3) connecting the microdialysis catheter (1) to the infusion reservoir (7) and to the collection reservoir (8) or the mixed reservoir (22) ;
- a microdialysis probe (1) comprising a first lumen (4) allowing the infusion fluid (11) to pass therethrough to a second lumen (5), with the second lumen (5) allowing discharge of the infusion fluid comprising the extracted compounds (12), and a microdialysis membrane (2) situated between the second lumen (5) and the exterior of the microdialysis probe (1), in contact with the biological fluid.

13. A dialysis system comprising an extraction device according to any one of the preceding claims, and such that it comprises at least:
- a probe with separate lumens (17), comprising a second catheter (5bis) allowing entry of biological fluid into the dialysis system and a first catheter (4bis) allowing exit of the biological fluid from the dialysis system;
- a reservoir (23) comprising i) the infusion fluid (11); ii) a dialysis compartment comprising a dialysis membrane (18) separating the infusion fluid (11) from the biological fluid, with the biological fluid entering the dialysis compartment via the second catheter (5bis) and exiting through the first catheter (4bis) therefrom.
